# EUROPEAN PATENT SPECIFICATION

(11) **EP 4 703 642 B1**
(45) Date of publication and mention of the grant of the patent: **26.08.2026**
(21) Application number: 24196439.4
(22) Date of filing: 26.08.2024
(51) Int. Cl.: F23N 5/26

(54) **COMPONENT WITH BUS TERMINATION**
BAUELEMENT MIT BUSABSCHLUSS
COMPOSANT AVEC TERMINAISON DE BUS

(43) Date of publication of application: 04.03.2026
(73) Proprietor: Siemens Aktiengesellschaft, 80333 München (DE)
(72) Inventor: Baader, Mathias, 76829 Landau (DE)
(74) Representative: Siemens Patent Attorneys

(56) References cited:
- EP-A1- 4 283 196

## Description

### Background

The present disclosure deals with a termination of a communication bus, wherein the communication bus is part of a combustion appliance. More specifically, the instant disclosure pertains to a sensor such as a flue gas sensor comprising the termination.

A combustion appliance comprises a burner and one or more conduits for supplying the burner with air and/or with fuel. The fuel can be a gaseous fuel and/or a liquid fuel such as oil. The fuel can be a fossil fuel. The fuel can also comprise hydrogen. One or more actuators control the supply of air and/or of fuel. For example, the combustion appliance can comprise a fan to supply the burner with air. The combustion appliance can also comprise one or more valves to control a supply of fuel to the burner.

In addition to these actuators, one or more sensors record signals associated with the combustion.

For example, an optical sensor such as an optical flame sensor can monitor the presence of a flame inside the burner. An optical sensor and a circuit for processing signals from the sensor are disclosed in the European patent EP3339736B1. An arrangement comprising a plurality of optical sensors is disclosed in the European patent EP3663646B1.

The patent EP3663646B1 also discloses an ionization electrode. The arrangement of EP3663646B1 comprises an ionization electrode and a first optical flame sensor. A controller processes the signals from these sensors and determines if a flame lift-off condition exists.

Combustion appliances also comprise exhaust conduits and flue gas sensors can be placed inside exhaust gas conduits. Flue gas sensors such as the flue gas sensor of the European patent application EP3783355A1 can be based on metal oxides such as zirconium dioxide. The patent application EP3783355A1 was filed on 26 May 2020 and claims a priority date of 21 August 2019.

A partial pressure of oxygen inside the sensor is determined based on two Nernst voltages. A signal indicative of oxygen in the exhaust gas of the combustion appliance of EP3783355A1 is recorded. A controller uses this signal to control combustion inside a combustion chamber of the combustion appliance.

Signals originating from the various sensors of the combustion appliance are processed by a controller of the combustion appliance. These signals can be analog signals such as signals in a range between four Milliamperes and twenty Milliamperes. The patent EP3339736B1 and the patent application EP3783355A1 disclose analog-to-digital converters and sigma-delta modulation to process such analog signals. More specifically, the European patent application EP2899548A1 and the European patent EP2899548B1 disclose detection circuits having analog-to-digital converters. The analog-to-digital converters of EP2899548A1 and of EP2899548B1 process input voltages of no more than two Volts. The detection circuits also comprise transformation circuits with only passive components.

The actuators and the sensors of a combustion appliance typically communicate with a system controller. The system controller can be arranged remotely from the actuators and from the sensors of the appliance. According to the European patent application EP0751350A2 and according to the European patent EP0751350B1, the combustion appliance can comprise a communication bus. The application EP0751350A2 was filed in 1996 and claims a priority of 29 June 1995.

The communication busses of EP0751350A2 and of EP0751350B1 are controller area network busses (CAN-busses) in accordance with the standard ISO/DS 11898. The transmission of signals via the CAN-busses of the combustion appliances of EP0751350A2 and of EP0751350B1 is message-oriented. The CAN-busses also afford reliable transmission of data over distances such as forty metres or one hundred metres.

The CAN-busses of EP0751350A2 and of EP0751350B1 connect a system controller to a burner unit. To that end, the system controller comprises an interface controller and the burner unit comprises a special-purpose controller. The CAN-busses of EP0751350A2 and of EP0751350B1 also connect a burner unit to a gas unit. The connection between the system controller and the burner unit is separate from the connection between the burner unit and the gas unit. The gas unit also comprises a special-purpose controller to connect to the CAN-bus.

The special-purpose controller of the burner unit comprises a plurality of input ports and a plurality of output ports. The special-purpose controller of the burner unit converts electric signals originating from an oil gauge and from a valve into digital signals. The digital signals are then forwarded via the CAN-bus.

Likewise, the special-purpose controller of the gas unit comprises a plurality of input ports and a plurality of output ports. The special-purpose controller of the gas unit converts signals originating from the bus into electric signals. The electric signals are then forwarded to various gas valves.

The European patent EP3301364B1 discloses a combustion appliance wherein a system controller connects to an anemometric sensor via a CAN-bus. The system controller also connects to a drive of a fan via the CAN-bus. The CAN-bus of EP3301364B1 can be a two-wires bus.

Communication busses such as controller area network busses can require impedances to terminate the busses. The impedances are often arranged at the ends of the busses and suppress reflections. The instant disclosure deals with a component of a combustion appliance that connects to a bus and provides a switchable termination.

### Summary

The invention deals with a component of a combustion appliance. The component comprises a bus termination that can be activated. The combustion appliance can be a commercial and/or residential and/or industrial combustion appliance having a burner and a heat consumer. The combustion appliance typically comprises a boiler such as a boiler for domestic hot water.

The component comprises a switchable electric impedance. The switchable and/or activatable electric impedance comprises an impedance such as a resistor. The impedance can be switched on thereby electrically and/or galvanically connecting it in between two bus wires. More specifically, the resistor can be switched on thereby electrically and/or galvanically connecting it in between two bus wires. The switchable and/or activatable electric impedance is then activated.

The impedance can be switched off thereby electrically and/or galvanically disconnecting it from at least one of the bus wires. More specifically, the resistor can be switched off thereby electrically and/or galvanically disconnecting it from at least one of the bus wires. The switchable and/or activatable electric impedance is then deactivated.

The bus wires comprise portions of bus wires of a controller area network bus. The bus wires can, by way of non-limiting example, comprise a portion of a HIGH bus wire of a controller area network bus. The two bus wires can also comprise a portion of a LOW bus wire of a controller area network bus. The bus wires can be portions of bus wires of a controller area network bus. The bus wires can, by way of non-limiting example, consist of the portion of the HIGH bus wire and of the portion of the LOW bus wire.

The component uses the bus such as the controller area network bus to communicate with a controller of the combustion appliance. The controller can comprise a system controller of the combustion appliance. The controller can be a system controller of the combustion appliance.

A tangible, electric interface is situated on an outer surface of the component. The tangible, electric interface is situated such that an operator and/or a user can access it. The tangible, electric interface breaks or makes an electric connection thereby terminating or not terminating the bus. More specifically, a controller area network bus is terminated or is not terminated.

The tangible, electric interface can, by way of non-limiting example, comprise a bridge connection and/or a jumper. When the bridge connection and/or the jumper are received by a socket, an electric connection exists inside the tangible, electric interface. The tangible, electric interface is in a short circuit condition. The bridge connection and/or the jumper can be part of a plug to be received by the socket.

The tangible, electric interface can, by way of non-limiting example, comprise a mechanical switch. When the mechanical switch closes, an electric connection exists inside the tangible, electric interface. The tangible, electric interface is in the short circuit condition. When the mechanical switch opens, an electric connection no longer exists inside the tangible, electric interface. The tangible, electric interface is in an open circuit condition. The mechanical switch can be part of a plug to be received by the socket.

A switching circuit is arranged in between the tangible, electric interface and the switchable and/or activatable impedance. The switching circuit electrically connects to the tangible, electric interface. The switching circuit is configured to read a status indicative of the electric connection inside the tangible, electric interface.

If the electric connection inside the tangible, electric interface opens, the switching circuit will activate the impedance. The bus such as the controller area network bus will then be terminated. If the electric connection inside the tangible, electric interface closes, the switching circuit will deactivate the impedance. The bus such as the controller area network bus will no longer be terminated.

In an alternate embodiment, the switching circuit will deactivate the impedance, if the electric connection inside the tangible, electric interface opens. The bus such as the controller area network bus will in this alternate embodiment not be terminated. The switching circuit will in this alternate embodiment activate the impedance, if the electric connection inside the tangible, electric interface closes. Consequently, the bus such as the controller area network bus will be terminated.

### Brief description of the drawings

Various features will become apparent to those skilled in the art from the following detailed description of the disclosed non-limiting embodiments. The drawings that accompany the detailed description can be briefly described as follows:
FIG 1 schematically illustrates a combustion appliance having a plurality of actuators and sensors and busses connected thereto.
FIG 2 schematically shows a first portion of a bus termination circuit.
FIG 3 schematically shows a second portion of a bus termination circuit.
FIG 4 schematically shows a third portion of a bus termination circuit.

### Detailed description

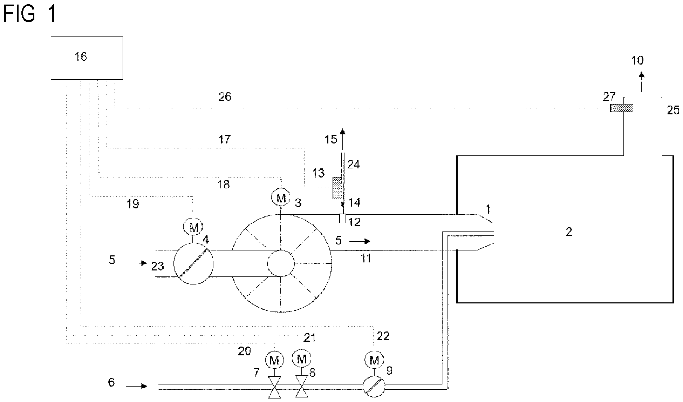

FIG 1 shows a system comprising a burner 1, a heat consumer 2, a fan 3 with a speed that can be set, and a damper 4 with motorized adjustment. The damper 4 with motorized adjustment is arranged after the air inlet 23. The heat consumer 2 (heat exchanger) can, for example, comprise a hot water vessel and/or a boiler. The heat consumer 2 can, for example, also be a boiler and/or a domestic hot water boiler and/or a residential hot water boiler.

The throughflow (particle flow and/or mass flow) 5 of the fluid air can be set in accordance with FIG 1 by the damper 4 with motorized adjustment. The throughflow 5 can also be set by specifying the rotational speed of the fan.

In the absence of the damper 4, the air throughflow 5 can also be adjusted just by setting the speed of the fan 3. Pulse-width modulation comes into consideration for adjusting the speed of the fan 3 for example. In accordance with another form of embodiment the motor of the fan 3 is connected to a converter. The speed of the fan 3 is thus adjusted via the frequency of the converter.

In accordance with another form of embodiment the fan runs at a fixed, invariable speed. The air throughflow 5 is then defined by the position of the damper 4. In addition, further actuators are possible, which change the air throughflow 5. In such cases an adjustment of the burner nozzle or of an adjustable flap in the waste gas vent duct can be involved.

The throughflow 6 (for example particle flow and/or mass flow) of the fluid fuel is set by a fuel damper 9. In accordance with one form of embodiment the fuel damper 9 is a valve such as a valve with motorized adjustment.

Combustible gases such as natural gas and/or propane gas and/or hydrogen are considered as fuel for example. A liquid fuel such as heating oil is, by way of non-limiting example, also considered as a fuel. In this case the damper 9 is replaced by an oil pressure regulator with motorized adjustment in the return of the oil nozzle. The safety shutdown function and/or closing function are implemented by the redundant safety valves 7, 8. In accordance with a specific form of embodiment the safety valves 7, 8 and the fuel damper 9 are realized as an integrated unit.

Fuel is mixed into the flow 5 of air in and/or before the burner 1. The mixture is burned in the combustion chamber of the heat consumer 2. The heat is transported onwards in the heat consumer 2. For example, heated water is taken away via a pump to heating elements and/or in industrial firing systems an item is heated (directly). The exhaust gas flow 10 is vented via an exhaust gas path 25, for example a chimney.

A system controller 16 coordinates all actuators so that the correct throughput 6 of fuel is set via the setting of the damper 9 for the corresponding air throughflow 5. Thus, the desired air-to-fuel ratio λ is produced. In accordance with a specific form of embodiment the system controller 16 is designed as a microcontroller. In accordance with another specific form of embodiment the system controller 16 is designed as a microprocessor.

To this end, the system controller 16 sets the fan 3 via the signal line 18 to the values stored in the system controller 16. Communication between the controller 16 and the fan 3 preferably involves a digital communication bus such as a CAN-bus implying that the signal line 18 is a logical path. Communication between the controller 16 and the fan 3 advantageously involves a digital communication protocol. The signal line 18 can, by way of non-limiting example, comprise a five-wire cable or a four-wire cable having a screen.

The air damper 4 is set via the signal line 19 to the values stored in the system controller 16. Communication between the controller 16 and the damper 4 preferably involves a digital communication bus such as a CAN-bus implying that the signal line 19 is a logical path. Communication between the controller 16 and the damper 4 advantageously involves a digital communication protocol. The signal line 19 can, by way of non-limiting example, comprise a five-wire cable or a four-wire cable having a screen.

Preferably the system controller 16 comprises a memory such as a non-volatile memory. Those values are stored in the memory. Ideally, those values are stored in the non-volatile memory. The setting of the fuel damper 9 is specified via the signal line 22. Communication between the controller 16 and the damper 9 preferably involves a digital communication bus such as a CAN-bus implying that the signal line 22 is a logical path. Communication between the controller 16 and the damper 9 advantageously involves a digital communication protocol. The signal line 22 can, by way of non-limiting example, comprise a five-wire cable or a four-wire cable having a screen.

In operation the safety shut-off valves 7, 8 are set via the signal lines 20, 21. Communication between the controller 16 and the valves 7, 8 preferably involves a digital communication bus such as a CAN-bus implying that the signal lines 20, 21 are logical paths. Communication between the controller 16 and the valves 7, 8 advantageously involves a digital communication protocol. The signal lines 20, 21 can, by way of non-limiting example, comprise a five-wire cable or a four-wire cable having a screen.

If faults are to be uncovered in a component selected from
- the damper 4,
- the damper 9,
- the fan 3,
then this can be done by a safety-related feedback via at least one of:
- the signal line 19 for the damper 4,
- the bidirectional signal line 19 for the damper 4,
- the signal line 22 for the damper 9,
- the bidirectional signal line 22 for the damper 9,
- the signal line 18 for the fan 3,
- the bidirectional signal line 18 for the fan 3.

A safety-related position message can be realized for example via redundant position generators. If a safety-related feedback about the rotational speed is required, this can be done via the (bidirectional) signal line 18 using speed sensors such as safety-related speed sensors. It is worth stressing that the term safety-related is defined in second edition of the standard IEC 61508-4 of April 2010.

Redundant speed sensors can be used for this purpose for example and/or the measured speed can be compared with required speed. The activation and feedback signals can be transferred via different signal lines and/or via a bidirectional bus. The bidirectional preferably comprises a digital, bidirectional bus. The bidirectional ideally is a digital, bidirectional bus.

Fitted before the burner is a side duct 24. A small amount 15 of outflowing air flows outwards through the side duct 24. Ideally the air flows out in this case into the space from which the fan 3 sucks in the air. In accordance with another form of embodiment the outflowing air flows out into the firing space of the heat consumer 2. In accordance with another form of embodiment the air flows back into the air duct 11. In this case a flow resistance element is arranged (at least locally) in the air duct 11 between tapping off point and return. The side duct 24, together with the burner 1 and the exhaust gas path 25 of the heat consumer 2, form a flow divider.

A flow resistance element such as an element in the form of a diaphragm 14 is fitted in the side duct 24. With the flow resistance element 14 the amount 15 of outflowing air of the flow divider is defined.

In accordance with a specific form of embodiment, the admittance surface of the flow resistance element 14 can be adjusted by a motor. To avoid and/or to remedy blockages caused by suspended particles, the admittance surface of the flow resistance element 14 can be adjusted. The flow resistance element 14 can be opened and/or can be closed. The admittance surface of the flow resistance element 14 is preferably adjusted multiple times to avoid and/or to remedy blockages.

With this arrangement the throughflow 15 (particle flow and/or mass flow) through the side duct 24 is a measure for the air flow 5 through the burner 1. In this case influences resulting from changes in the density of the air for example are compensated for by changes in the absolute pressure. These influences can also be compensated by the air temperature through the mass flow sensor 13. Normally the flow 15 is very much smaller than the air flow 5. In accordance with a specific form of embodiment the (particle and/or mass) flow 15 through the side duct 24 is smaller by at least a factor of one hundred, preferably by at least a factor of one thousand, further preferably by at least a factor of ten thousand than the (particle and/or mass) flow 5 through the air duct 11.

Sensors such as the mass flow sensor 13 allow measurement at high flow speeds, specifically in conjunction with combustion devices in operation. Typical values of such flow speeds lie in ranges between 0.1 metres per second and five metres per second, ten metres per second, or twenty metres per second. Typical values of such flow speeds can also lie in ranges between 0.1 metres per second and fifty metres per second, or even one hundred metres per second. Mass flow sensors that are suitable for the present disclosure are for example OMRON^{®} D6F-W or SENSOR TECHNICS^{®} WBA-type sensors. The usable range of these sensors typically begins at speeds between 0.01 metres per second and 0.1 metres per second and ends at a speed of for example five metres per second. The usable range of these sensors can also end at ten metres per second, fifteen metres per second, twenty metres per second, or even one hundred metres per second. In other words, lower limits such as 0.1 metres per second can be combined with upper limits such as five metres per second or ten metres per second. Lower limits such as 0.1 metres per second can also be combined with upper limits such as fifteen metres per second, or twenty metres per second. Lower limits such as 0.1 metres per second can even be combined with upper limits such as one hundred metres per second.

Communication between the controller 16 and the sensor 13 preferably involves a digital communication bus such as a CAN-bus. Communication between the controller 16 and the sensor 13 advantageously involves a digital communication protocol. The signal line 17 can, by way of non-limiting example, comprise a five-wire cable or a four-wire cable having a screen. The signal line 17 can also indicate a logical path.

A flue gas sensor 27 can be arranged inside or adjacent the exhaust gas path 25. In a special embodiment, the flue gas sensor 27 is arranged inside and/or adjacent a chimney. Details of a sensor such as the flue gas sensor 27 are disclosed in the aforementioned application EP3783355A1.

Communication between the system controller 16 and the flue gas sensor 27 preferably involves a digital communication bus such as a CAN-bus. Communication between the controller 16 and the flue gas sensor 27 advantageously involves a digital communication protocol. The signal line 26 can, by way of non-limiting example, comprise a five-wire cable or a four-wire cable having a screen. The signal line 26 can also indicate a logical path.

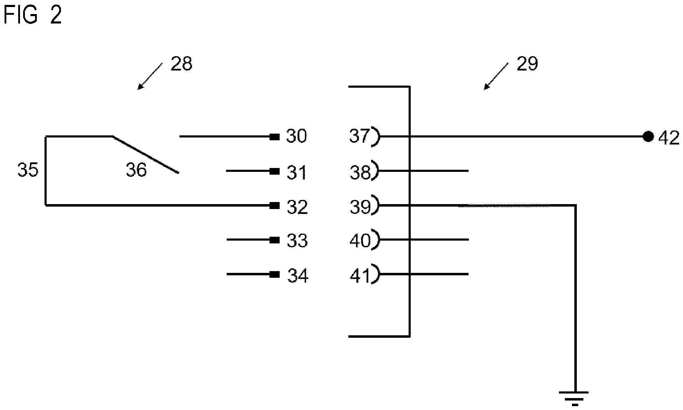

Now turning to FIG 2, a plug 28 and a socket 29 are illustrated. The plug 28 comprises a plurality of pins. The plug 28 preferably comprises five pins. These pins are labelled with reference numerals 30 to 34 in FIG 2.

A bridge connection 35 extends from the first pin 30 to the third pin 32 of the plug 28. The bridge connection 35 advantageously comprises a wired connection such as a connection made out of copper wire. In a special embodiment, the bridge connection 35 is a wired connection such as a connection made out of copper wire.

The bridge connection 35 optionally comprises a switch 36 having an open position and a closed position. The switch 36 can, by way of non-limiting example, comprise at least one of:
- a mechanical switch 36,
- a dual in-line package switch.

In a special embodiment, the switch 36 is selected from
- a mechanical switch 36,
- a dual in-line package switch.

In another special embodiment, the bridge connection 35 comprises a wired connection such as a copper wire and a switch 36.

When the switch 36 is in its closed position, a short circuit condition exists between the first pin 30 and the third pin 32. That is, the electric resistance between the first pin 30 and the third pin 32 at temperatures of 293 Kelvins is less than 1 Ohm. The electric resistance between the first pin 30 and the third pin 32 preferably is less than 0.5 Ohm or even less than 0.2 Ohm.

When the switch 36 is in its open position, the first pin 30 and the third pin 32 are electrically isolated. That is, the electric resistance between the first pin 30 and the third pin 32 at temperatures of 293 Kelvins is more than two MegaOhms. The electric resistance between the first pin 30 and the third pin 32 preferably is more than five MegaOhms or even more than ten MegaOhms.

It is envisaged that the plug 28 can mechanically fit into the socket 29. The plug 28 can also be mechanically removed from the socket 29. The socket 29 can, by way of non-limiting example, be situated on an outer surface of a component of a combustion appliance such as
- a fan 3,
- an actuator of the fan 3,
- a damper 4,
- an actuator of the damper 4,
- a valve 7 - 9,
- an actuator of one of the valves 7 - 9,
- a flow sensor 13,
- a mass flow sensor 13,
- a flue gas sensor 27.

The plug 28 can then fit into the socket 29 mounted on the outer surface of the component. The plug 28 can also be mechanically removed from the socket 29 mounted on the outer surface of the component. If the bridge connection 35 comprises no switch 36, the plug 28 can suffice to electrically connect the first pin 30 to the third pin 32.

It is still envisaged that the plug 28 comprises a jumper. When the jumper is attached to the socket 29, the jumper electrically connects the first pin 30 to the third pin 32.

If the bridge connection 35 comprises a switch 36 such as a mechanical switch, the switch 36 can be situated on an outer surface of a component of a combustion appliance. The component can be selected from at least one of:
- a fan 3,
- an actuator of the fan 3,
- a damper 4,
- an actuator of the damper 4,
- a valve 7 - 9,
- an actuator of one of the valves 7 - 9,
- a flow sensor 13,
- a mass flow sensor 13,
- a flue gas sensor 27.

The above lists of components of a combustion appliance are not exhaustive.

The socket 29 comprises a plurality of connectors. The socket 29 preferably comprises five connectors. These connectors are labelled with reference numerals 37 to 41 in FIG 2. A first connector 37 is configured to receive the first pin 30, thereby electrically connecting the first pin 30 to the first connector 37. That is, the electric resistance between the first pin 30 and the first connector 37 at temperatures of 293 Kelvins is less than 1 Ohm. The electric resistance between the first pin 30 and the first connector 37 preferably is less than 0.5 Ohm or even less than 0.2 Ohm. A third connector 39 is configured to receive the third pin 32, thereby electrically connecting the third pin 30 to the third connector 39. That is, the electric resistance between the third pin 32 and the third connector 39 at temperatures of 293 Kelvins is less than 1 Ohm. The electric resistance between the third pin 32 and the third connector 39 preferably is less than 0.5 Ohm or even less than 0.2 Ohm.

Preferably, a second connector 38 of the socket 29 is configured to receive the second pin 31 of the plug 28. Likewise, a fourth connector 40 of the socket 29 is configured to receive the fourth pin 33 of the plug 28. Likewise, a fifth connector 41 of the socket 29 is configured to receive the fifth pin 34 of the plug 28.

The third connector 39 electrically connects to ground and the first connector 37 electrically connects to a terminal 42. More specifically, the third connector 39 galvanically connects to ground and the first connector 37 galvanically connects to a terminal 42. If the bridge connection 35 comprises no switch 36, an attachment of the plug 28 to the socket 29 will connect the terminal 42 to ground.

If the bridge connection 35 comprises a switch 36 and the switch 36 is in its closed position, the terminal 42 will also connect to ground. More specifically, the terminal 42 will galvanically connect to ground. If the switch 36 comprises a mechanical switch such as a dual in-line switch and the mechanical switch closes, the terminal 42 will electrically connect to ground. If the switch 36 is a mechanical switch such as a dual in-line switch and the mechanical switch closes, the terminal 42 will electrically connect to ground. In these embodiments, the plug 28 can be permanently attached to the socket 29. The plug 28 and the socket 29 can be unitary.

Whenever the terminal 42 connects to ground, a short circuit condition exists between the terminal 42 and ground. That is, the electric resistance between the terminal 42 and ground at temperatures of 293 Kelvins is less than three Ohms. The electric resistance between the terminal 42 and ground preferably is less than 1.5 Ohm or even less than 0.6 Ohm.

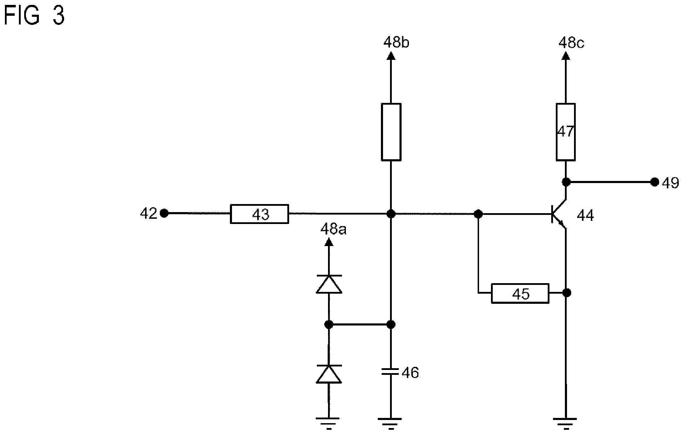

Now referring to FIG 3, a NOT gate is disclosed. The NOT gate as shown in FIG 3 connects to circuit shown in FIG 2 as indicated by the terminal 42. In a special embodiment, the circuit as shown in FIG 2 galvanically connects to the circuit as shown in FIG 3 via terminal 42. The circuits in FIG 2 and in FIG 3 can still galvanically connect without a terminal.

An electric resistor 43 protects the circuit from disturbances such as electrostatic discharges. The resistor 43 can, by way of non-limiting examples, exhibit an electric resistivity at a temperature of 293 Kelvins of five hundred Ohms or two thousand Ohms. The resistor 43 can, by way of another non-limiting example, exhibit an electric resistivity at a temperature of 293 Kelvins of one thousand Ohms. The switch 44 advantageously comprises a controllable switch and can be controlled via a signal at terminal 42. The switch 44 can, by way of non-limiting example, comprise a transistor. The switch 44 ideally is a controllable switch and can be controlled via a signal at terminal 42. The switch 44 can, by way of non-limiting example, be a transistor.

Another electric resistor 45 mitigates adverse effects of electric currents caused by disturbances and/or leakages. The resistor 45 can, by way of non-limiting examples, exhibit an electric resistivity at a temperature of 293 Kelvins between fifty Kiloohms and two hundred Kiloohms. The resistor 45 can also exhibit an electric resistivity of one hundred Kiloohms or of substantially one hundred Kiloohms.

A capacitor 46 also protects the circuit from disturbances such as electrostatic discharges. The capacitor 46 can, by way of non-limiting examples, exhibit an electric capacitance at a temperature of 293 Kelvins between fifty Nanofarads and two hundred Nanofarads. The capacitor 46 can also exhibit an electric capacitance of one hundred Nanofarads.

Another resistor 47 limits electric currents through the switch 44. The resistor 47 can, by way of non-limiting examples, exhibit an electric resistivity at a temperature of 293 Kelvins between five Kiloohms and twenty Kiloohms. The resistor 47 can also exhibit an electric resistivity of ten Kiloohms.

The electric voltages at the terminals 48a - 48c are chosen such that an output voltage at the output terminal 49 is commensurate with a logical HIGH signal. It is envisaged that same voltages are applied at least two of the terminals 48a - 48c. More preferably, same voltages are applied to all the terminals 48a - 48c.

In an embodiment, an electric voltage between two Volts and five Volts is applied to the supply terminal 48c. More preferably, an electric voltage between 2.7 Volts and five Volts is applied to the supply terminal 48c. Still more preferably, an electric voltage of 3.3 Volts is applied to the supply terminal 48c.

In a related embodiment, electric voltages between two Volts and five Volts are applied to the terminals 48a - 48c. More preferably, electric voltages between 2.7 Volts and five Volts are applied to the terminals 48a - 48c. Still more preferably, electric voltages of 3.3 Volts are applied to the terminals 48a - 48c.

The circuit as shown in FIG 3 is a NOT gate. That is, a voltage of zero Volts at the input terminal 42 causes the switch 44 to not conduct electricity or to substantially not conduct electricity. More specifically, voltage of zero Volts at the input terminal 42 causes the transistor 44 to not conduct electricity or to substantially not conduct electricity. Where the switch 44 and/or the transistor 44 do substantially not conduct electricity, negligible electric currents such as leakage currents can still occur. The voltage at the output terminal 49 then becomes a logical HIGH signal such as 3.3 Volts. More specifically, a voltage of zero Volts at the input terminal 42 causes the transistor 44 to not conduct electricity. The voltage at the output terminal 49 then becomes a logical HIGH signal such as 3.3 Volts. A voltage of 3.3 Volts at the input terminal 42 is indicative of a logical HIGH and causes the switch 44 to conduct electricity. The voltage at the output terminal 49 then becomes a logical LOW signal such as zero Volts or substantially zero Volts. More specifically, a voltage of 3.3 Volts at the input terminal 42 is indicative of a logical HIGH and causes the transistor 44 to conduct electricity. The voltage at the output terminal 49 then becomes a logical LOW signal such as zero Volts or substantially zero Volts. In this context, substantially zero Volts means a signal that is recognised as a logical LOW signal.

It is worth stressing that a NOT gate can be implemented in other ways. The circuit as shown in FIG 3 is exemplary. The instant disclosure is not limited to the NOT gate as illustrated in FIG 3.

Effectively, a conductive path between the first connector 37 and the third connector 39 causes a logical HIGH such as 3.3 Volts at the output terminal 49. The conductive path can, by way of non-limiting example, be afforded by a plug 28 that is inserted in the socket 29. This assumes that the plug 28 does not provide a switch 36. The conductive path can, by way of non-limiting example, be afforded by a switch 36 in its closed position. This assumes that an entity such as the plug 28 provides a switch 36 such as a mechanical switch.

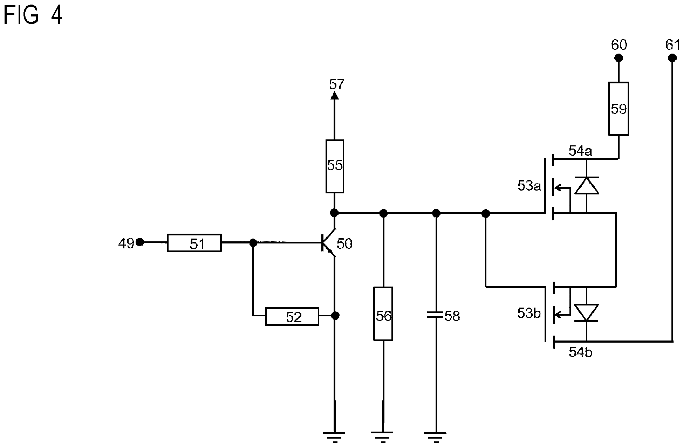

For a controller area network bus to terminate correctly, the logic signal at the output terminal 49 shown in FIG 3 needs to cause such a termination. The same is illustrated in FIG 4.

If a voltage indicative of a logical LOW signal is applied at the terminal 49 in FIG 4, the switch 50 will not or will substantially not conduct electricity. In an embodiment, the transistor 50 will not or will substantially not conduct electricity. Where the switch 50 and/or the transistor 50 do substantially not conduct electricity, negligible electric currents such as leakage currents can still occur. If any voltage other than zero Volts is applied to the terminal 49, an electric resistor 51 will limit electric currents through the switch 50. More specifically, an electric resistor 51 will limit electric currents through the transistor 50.

The resistor 51 can, by way of non-limiting examples, exhibit an electric resistivity at a temperature of 293 Kelvins between fifty Kiloohms and two hundred Kiloohms. The resistor 51, by way of another non-limiting example, exhibit an electric resistivity at a temperature of 293 Kelvins of one hundred Kiloohms or substantially one hundred Kiloohms.

Another electric resistor 52 mitigates adverse effects of electric currents caused by disturbances and/or leakages. The resistor 52 can, by way of non-limiting examples, exhibit an electric resistivity at a temperature of 293 Kelvins between fifty Kiloohms and two hundred Kiloohms. The resistor 52 can also exhibit an electric resistivity of one hundred Kiloohms or substantially one hundred Kiloohms.

The voltage at the control terminal 53a, 53b of the switch 54a, 54b is thus determined by the state of the switch 50. Where the switch 50 does not or does substantially not conduct electricity, a voltage divider is formed by the resistors 55 and 56. A plethora of considerations is factored in when choosing the resistors 55 and 56. These include, but are not limited to, the voltage levels at terminals 60 and 61 as well es permissible voltages at the gates 53a and 53b.

It is envisaged that the electric resistivity of the resistor 56 is twice the electric resistivity of the resistor 55. For example, the electric resistor 55 can exhibit an electric resistivity of fifty Kiloohms and the electric resistor 56 can exhibit an electric resistivity of one hundred Kiloohms. The electric resistor 55 can also exhibit an electric resistivity of one hundred Kiloohms and the electric resistor 56 can exhibit an electric resistivity of two hundred Kiloohms. The electric resistor 55 can still exhibit an electric resistivity of two hundred Kiloohms and the electric resistor 56 can exhibit an electric resistivity of four hundred Kiloohms. These values of electric resistivity are exemplary and are applicable to room temperature such as 293 Kelvins.

A voltage at the supply terminal 57 of twenty-four Volts will thus lead to a voltage of sixteen Volts at the control terminal 53a, 53b. This assumes that the switch 50 does not or does substantially not conduct electricity. More specifically, this assumes that the transistor 50 does not or does substantially not conduct electricity.

Where the voltage at the supply terminal 57 is different from twenty-four Volts, a different voltage divider 55, 56 can be used. For example, the voltage at the supply terminal 57 can be thirty-two Volts. A voltage divider 55, 56 with equal values of resistivity of the resistors 55 and 56 is then employed to ascertain a voltage of sixteen volts at the control terminal 53a, 53b. This assumes that the switch 50 does not or does substantially not conduct electricity. More specifically, this assumes that the transistor 50 does not or does substantially not conduct electricity.

A voltage of thirty-two volts at terminal 57 can, by way of non-limiting example, result in resistors 55, 56 that exhibit electric resistivities of one hundred Kiloohms. The resistors 55 and 56 can also each exhibit resistivities of substantially one hundred Kiloohms. A voltage of thirty-two volts at terminal 57 can also result in resistors 55, 56 that each exhibit electric resistivities of two hundred Kiloohms. The resistors 55 and 56 can also each exhibit resistivities of substantially two hundred Kiloohms. A voltage of thirty-two volts at terminal 57 can also result in resistors 55, 56 that each exhibit electric resistivities of five hundred Kiloohms. The resistors 55 and 56 can also each exhibit resistivities of substantially five hundred Kiloohms. These values of electric resistivity are exemplary and are applicable to room temperature such as 293 Kelvins. These values of electric resistivity are chosen such that electric currents through the voltage divider 55, 56 do not become excessive.

Where the voltage at the supply terminal 57 is different from twenty-four Volts, a different voltage divider 55, 56 can be used. For example, the voltage at the supply terminal 57 can be forty-eight Volts. The electric resistivity of the resistor 55 is then twice or substantially twice the electric resistivity of the resistor 56.

A voltage of forty-eight volts at terminal 57 can, by way of non-limiting example, result in a resistor 55 that has an electric resistivity of two hundred Kiloohms. The resistor 56 will then have an electric resistivity of one hundred Kiloohms. Also, the resistor 55 can have a resistivity of substantially two hundred Kiloohms and the resistor 56 can have a resistivity of substantially one hundred Kiloohms. A voltage of forty-eight volts at terminal 57 can also result in a resistor 55 that has an electric resistivity of one hundred Kiloohms. The resistor 56 will then have an electric resistivity of fifty Kiloohms. Also, the resistor 55 can have a resistivity of substantially one hundred Kiloohms and the resistor 56 can have a resistivity of substantially fifty Kiloohms. A voltage of forty-eight volts at terminal 57 can still result in a resistor 55 that has an electric resistivity of four hundred Kiloohms. The resistor 56 will then have an electric resistivity of two hundred Kiloohms. Also, the resistor 55 can have a resistivity of substantially four hundred Kiloohms and the resistor 56 can have a resistivity of substantially two hundred Kiloohms. These values of electric resistivity are exemplary and are applicable to room temperature such as 293 Kelvins. These values of electric resistivity are chosen such that electric currents through the voltage divider 55, 56 do not become excessive.

The above lists of voltages at the terminal 57 and of voltage dividers 55, 56 are not exhaustive.

The resistors 55, 56 together with the capacitor 58 form a resistive capacitive circuit. The electric resistivities of the resistors 55, 56 and the capacity of the capacitor 58 define a time constant for changing signals at the control terminal 53a, 53b. The capacitor 58 can, by way of non-limiting examples, exhibit an electric capacitance at a temperature of 293 Kelvins between fifty Nanofarads and two hundred Nanofarads. The capacitor 58 can also exhibit an electric capacitance of one hundred Nanofarads.

An electric voltage of sixteen Volts applied at the control terminal 53a, 53b causes the switch 54a, 54b to conduct electricity. The electric resistivity of the path between the terminals 60 and 61 is then essentially determined by the resistivity of the impedance 59.

The terminal 60 can, by way of non-limiting example, connect to the HIGH portion of a controller area network bus. Likewise, the terminal 61 can connect to the LOW portion of a controller area network bus. When the switch 54a, 54b conducts electricity, an electric current will flow from the HIGH terminal 60 to the LOW terminal 61. This electric current is determined or is substantially determined by the impedance 59. In this context, the electric current is substantially determined by the impedance 59 because the switch 54a, 54b in its conducting state can also be an impedance.

It is envisaged that the impedance 59 comprises a resistor. An electric current between the HIGH terminal 60 and the LOW terminal 61 is then influenced by the resistivity of the resistor 59. It is still envisaged that the impedance 59 is a resistor. An electric current between the HIGH terminal 60 and the LOW terminal 61 is then determined or substantially determined by the resistor 59. In this context, the electric current is substantially determined by the resistor 59 because the switch 54a, 54b in its conducting state can also be an impedance.

If a voltage indicative of a logical HIGH signal is applied at the terminal 49 in FIG 4, the switch 50 will conduct electricity. In an embodiment, the transistor 50 will conduct electricity. If a voltage of 3.3 Volts or of substantially 3.3 Volts is applied at the terminal 49 in FIG 4, the switch 50 will conduct electricity. In an embodiment, the transistor 50 will conduct electricity.

The voltage at the control terminal 53a, 53b of the switch 54a, 54b is determined by the state of the switch 50. Where the switch 50 does conduct electricity, the voltage at the control terminal 53a, 53b of the switch 54a, 54b will be zero or substantially zero. The voltage at the control terminal 53a, 53b can be substantially zero because the switch 50 in its conducting state also has a limited resistivity. That limited resistivity is, however, small or negligible compared to the resistivity of the resistor 55. In an embodiment, a transistor 50 in its conducting state has a limited resistivity. That limited resistivity is, however, small or negligible compared to the resistivity of the resistor 55.

A voltage of zero or of substantially zero at the control terminal 53a, 53b causes the switch 54a, 54b to open. The electric resistivity of the path between the terminals 60 and 61 is then effectively determined by the impedance of the switch 54a, 54b in its opened state. The impedance of the switch 54a, 54b in its opened state is large compared to the impedance 59. Where the switch 54a, 54b opens, the connection between the terminals 60 and 61 effectively is an open circuit. A controller area network bus connected to the terminals 60 and 61 is not terminated by the impedance 59. That is, any termination of a controller area network bus connected to the terminals 60 and 61 does not involve the impedance 59.

In an embodiment, the electric resistivity of the path between the terminals 60 and 61 is then effectively determined by the electric resistivity of the switch 54a, 54b in its opened state. However, the electric resistivity of the switch 54a, 54b in its opened state is large compared to the electric resistivity of the resistor 59. Where the switch 54a, 54b opens, the connection between the terminals 60 and 61 effectively is an open circuit. A controller area network bus connected to the terminals 60 and 61 is not terminated by the resistor 59. That is, any termination of a controller area network bus connected to the terminals 60 and 61 does not involve the resistor 59.

In the exemplary embodiment shown in FIG 4, the control terminal 53a, 53b and the switch 54a, 54b comprise two anti-serial metal-oxide field-effect transistors. Ideally, the control terminal 53a, 53b and the switch 54a, 54b consist of two anti-serial metal-oxide field-effect transistors. In another embodiment, the control terminal 53a, 53b and the switch 54a, 54b comprise one or more electric relays. It can be necessary to provide a driver circuit to operate the one or more electric relays of the switch 54a, 54b.

In summary, a closed electric connection between the first connector 37 and the third connector 39 results in a LOW signal at the terminal 42. A LOW signal at the terminal 42 causes a HIGH signal at the terminal 49 of the NOT gate. The HIGH signal at the terminal 49 causes the switches 50 and 54a, 54b to deactivate the impedance 59. A controller area network bus between terminals 60 and 61 is not terminated.

An open connection between the first connector 37 and the third connector 39 results in a HIGH signal at the terminal 42. A HIGH signal at the terminal 42 causes a LOW signal at the terminal 49 of the NOT gate. The LOW signal at the terminal 49 causes the switches 50 and 54a, 54b to activate the impedance 59. A controller area network bus between terminals 60 and 61 is then terminated.

In a practical embodiment, the impedance 59 at a temperature of 293 Kelvins has an electric resistivity that matches the characteristic impedance of the bus. It is envisaged that the impedance 59 has an electric resistivity of substantially 120 Ohms. In a special embodiment, the impedance 59 at a temperature of 293 Kelvins has an electric resistivity of 120 Ohms.

As described in detail herein, the present invention deals with a component (3, 4, 7 - 9, 13, 27) for a combustion appliance, the component (3, 4, 7 - 9, 13, 27) comprising an outer surface and at least one tangible, electric interface (30, 32, 35, 36) configured to be electrically connected by a user such that the at least one tangible, electric interface (30, 32, 35, 36) is in a short circuit condition and configured to be electrically disconnected by the user such that the at least one tangible, electric interface (30, 32, 35, 36) is in an open circuit condition;
wherein the at least one tangible, electric interface (30, 32, 35, 36) is arranged on the outer surface of the component (3, 4, 7 - 9, 13, 27) such that the at least one tangible, electric interface (30, 32, 35, 36) is accessible to the user;
the component (3, 4, 7 - 9, 13, 27) also comprising at least one first bus wire and at least one second bus wire, wherein the at least one first bus wire is different from the at least one second bus wire;
the component (3, 4, 7 - 9, 13, 27) also comprising at least one switching circuit (29, 37 - 61), wherein the at least one switching circuit (29, 37 - 61) comprises at least one switchable electric impedance (54a, 54b, 59);
wherein the at least one switchable electric impedance (54a, 54b, 59) electrically connects to the at least one first bus wire;
wherein the at least one switching circuit (29, 37 - 61) is configured to:
   read from the at least one tangible, electric interface (30, 32, 35, 36) a status signal indicative of the open circuit condition or of the short circuit condition;
   process the status signal; and
   if the status signal indicates the open circuit condition:
      either activate the at least one switchable electric impedance (54a, 54b, 59) such that the at least one switchable electric impedance (54a, 54b, 59) electrically connects to the at least one second bus wire, thereby electrically connecting the at least one first bus wire to the at least one second bus wire via the at least one switchable electric impedance (54a, 54b, 59), or
      deactivate the at least one switchable electric impedance (54a, 54b, 59) such that the at least one switchable electric impedance (54a, 54b, 59) electrically disconnects from the at least one second bus wire, thereby electrically disconnecting the at least one first bus wire from the at least one second bus wire.

The at least one first bus wire can be separate from the at least one second bus wire. Any electric signal propagating via the at least one first bus wire electrically refers to a ground potential. Likewise, any electric signal propagating via the at least one second bus wire electrically refers to the ground potential.

It is envisaged that any of the aforementioned components (3, 4, 7 - 9, 13, 27) can also be a component (3, 4, 7 - 9, 13, 27) of the combustion appliance.

The at least one tangible, electric interface (30, 32, 35, 36) may comprise at least one electric connector (30, 32, 35, 36). The at least one tangible, electric interface (30, 32, 35, 36) may be at least one electric connector (30, 32, 35, 36). The at least one switching circuit (29, 37 - 61) may comprise at least one electronic switching circuit (29, 37 - 61). The at least one switching circuit (29, 37 - 61) may be at least one electronic switching circuit (29, 37 - 61). The at least one switchable electric impedance (54a, 54b, 59) may comprise at least one switchable electric impedance circuit (54a, 54b, 59). The at least one switchable electric impedance (54a, 54b, 59) may be at least one switchable electric impedance circuit (54a, 54b, 59).

It is envisaged that the at least one tangible, electric interface (30, 32, 35, 36) comprises at least one electric connector (30, 32, 35, 36) and that the at least one switching circuit (29, 37 - 61) comprises at least one electronic switching circuit (29, 37 - 61). It is still envisaged that the at least one tangible, electric interface (30, 32, 35, 36) is at least one electric connector (30, 32, 35, 36) and that the at least one switching circuit (29, 37 - 61) is at least one electronic switching circuit (29, 37 - 61).

It is envisaged that the at least one switching circuit (29, 37 - 61) comprises at least one electronic switching circuit (29, 37 - 61) and that the at least one switchable electric impedance (54a, 54b, 59) comprises at least one switchable electric impedance circuit (54a, 54b, 59). It is still envisaged that the at least one switching circuit (29, 37 - 61) is at least one electronic switching circuit (29, 37 - 61) and that the at least one switchable electric impedance (54a, 54b, 59) is at least one switchable electric impedance circuit (54a, 54b, 59).

It is envisaged that the at least one tangible, electric interface (30, 32, 35, 36) comprises at least one electric connector (30, 32, 35, 36) and that the at least one switchable electric impedance (54a, 54b, 59) comprises at least one switchable electric impedance circuit (54a, 54b, 59). It is still envisaged that the at least one tangible, electric interface (30, 32, 35, 36) is at least one electric connector (30, 32, 35, 36) and that the at least one switchable electric impedance (54a, 54b, 59) is at least one switchable electric impedance circuit (54a, 54b, 59).

The instant disclosure also deals with any of the components (3, 4, 7 - 9, 13, 27) as described above, wherein the at least one switching circuit (29, 37 - 61) is configured to:
if the status signal indicates the short circuit condition:
deactivate the at least one switchable electric impedance (54a, 54b, 59) such that the at least one switchable electric impedance (54a, 54b, 59) electrically disconnects from the at least one second bus wire, thereby electrically disconnecting the at least one first bus wire from the at least one second bus wire.

The present disclosure still deals with any of the components (3, 4, 7 - 9, 13, 27) as described above, wherein the at least one switching circuit (29, 37 - 61) comprises at least one NOT gate (42 - 49) electrically connected to the at least one tangible, electric interface (30, 32, 35, 36), wherein the at least one NOT gate (42 - 49) is configured to:
read from the at least one tangible, electric interface (30, 32, 35, 36) a status signal indicative of the open circuit condition or of the short circuit condition;
process the status signal; and
if the status signal indicates the open circuit condition:
   generate a logical LOW output signal.

The instant disclosure also deals with any of the components (3, 4, 7 - 9, 13, 27) as described above and involving a logical LOW signal, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and to the at least one electric resistor (59), wherein the at least one first switch (50) is configured to:
read the logical LOW output signal from the at least one NOT gate;
process the logical LOW signal;
transmit the processed logical signal to the at least one second switch (54a, 54b);
wherein the at least one second switch (54a, 54b) is configured to:
read the processed logical signal from the at least one first switch (50); and
electrically connect the at least one electric resistor (59) to the at least one second bus wire.

The present disclosure still deals with any of the components (3, 4, 7 - 9, 13, 27) as described above and involving a logical LOW signal, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and to the at least one electric resistor (59), wherein the at least one electric resistor (59) electrically connects to the at least one first bus wire, wherein the at least one first switch (50) is configured to:
read the logical LOW output signal from the at least one NOT gate;
process the logical LOW signal;
transmit the processed logical signal to the at least one second switch (54a, 54b);
wherein the at least one second switch (54a, 54b) is configured to:
read the processed logical signal from the at least one first switch (50); and
electrically connect the at least one electric resistor (59) to the at least one second bus wire.

The instant disclosure still deals with any of the components (3, 4, 7 - 9, 13, 27) as described above and involving a logical LOW signal, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and galvanically connects to the at least one electric resistor (59), wherein the at least one electric resistor (59) galvanically connects to the at least one first bus wire, wherein the at least one first switch (50) is configured to:
read the logical LOW output signal from the at least one NOT gate;
process the logical LOW signal;
transmit the processed logical signal to the at least one second switch (54a, 54b);
wherein the at least one second switch (54a, 54b) is configured to:
read the processed logical signal from the at least one first switch (50); and
electrically connect the at least one electric resistor (59) to the at least one second bus wire.

The instant disclosure also deals with any of the aforementioned components (3, 4, 7 - 9, 13, 27) involving a logical LOW signal and at least one first switch (50), wherein the at least one first switch (50) is configured to electronically process the logical LOW signal.

The present disclosure still deals with any of the components (3, 4, 7 - 9, 13, 27) as described above, wherein the at least one switching circuit (29, 37 - 61) comprises at least one NOT gate (42 - 49) electrically connected to the at least one tangible, electric interface (30, 32, 35, 36), wherein the at least one NOT gate (42 - 49) is configured to:
read from the at least one tangible, electric interface (30, 32, 35, 36) a status signal indicative of the open circuit condition or of the short circuit condition;
process the status signal; and
if the status signal indicates the short circuit condition:
   generate a logical HIGH output signal.

The instant disclosure also deals with any of the aforementioned components (3, 4, 7 - 9, 13, 27) involving at least one NOT gate (42 - 49), wherein the at least one NOT gate (42 - 49) is configured to electronically process the status signal.

The present disclosure still deals with any of the components (3, 4, 7 - 9, 13, 27) as described above and involving a logical HIGH signal, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and to the at least one electric resistor (59), wherein the at least one first switch (50) is configured to:
read the logical HIGH output signal from the at least one NOT gate;
process the logical HIGH signal;
transmit the processed logical signal to the at least one second switch (54a, 54b);
wherein the at least one second switch (54a, 54b) is configured to:
read the processed logical signal from the at least one first switch (50); and
electrically disconnect the at least one electric resistor (59) from the at least one second bus wire.

The instant disclosure also deals with any of the components (3, 4, 7 - 9, 13, 27) as described above and involving a logical HIGH signal, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and to the at least one electric resistor (59), wherein the at least one electric resistor (59) electrically connects to the at least one first bus wire, wherein the at least one first switch (50) is configured to:
read the logical HIGH output signal from the at least one NOT gate;
process the logical HIGH signal;
transmit the processed logical signal to the at least one second switch (54a, 54b);
wherein the at least one second switch (54a, 54b) is configured to:
read the processed logical signal from the at least one first switch (50); and
electrically disconnect the at least one electric resistor (59) from the at least one second bus wire.

The present disclosure still deals with any of the components (3, 4, 7 - 9, 13, 27) as described above and involving a logical HIGH signal, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and galvanically connects to the at least one electric resistor (59), wherein the at least one electric resistor (59) galvanically connects to the at least one first bus wire, wherein the at least one first switch (50) is configured to:
read the logical HIGH output signal from the at least one NOT gate;
process the logical HIGH signal;
transmit the processed logical signal to the at least one second switch (54a, 54b);
wherein the at least one second switch (54a, 54b) is configured to:
read the processed logical signal from the at least one first switch (50); and
electrically disconnect the at least one electric resistor (59) from the at least one second bus wire.

The instant disclosure also deals with any of the aforementioned components (3, 4, 7 - 9, 13, 27) involving a logical HIGH signal and at least one first switch (50), wherein the at least one first switch (50) is configured to electronically process the logical HIGH signal.

The instant disclosure also deals with any of the aforementioned components (3, 4, 7 - 9, 13, 27) involving a processed logical signal, wherein the at least one second switch (54a, 54b) comprises at least one control terminal (53a, 53b), wherein the at least one second switch (54a, 54b) is configured to:
read the processed logical signal from the at least one first switch (50) via the at least one control terminal (53a, 53b).

The present disclosure also deals with any of the aforementioned components (3, 4, 7 - 9, 13, 27) involving a processed logical signal, wherein the at least one first switch (50) comprises a collector terminal;
wherein the at least one second switch (54a, 54b) comprises at least one control terminal (53a, 53b);
wherein the collector terminal electrically connects to the at least one control terminal (53a, 53b); and
wherein the at least one second switch (54a, 54b) is configured to:
   read the processed logical signal from the at least one first switch (50) via the at least one control terminal (53a, 53b).

The present disclosure also deals with any of the aforementioned components (3, 4, 7 - 9, 13, 27) involving a processed logical signal, wherein the at least one first switch (50) comprises a collector terminal;
wherein the at least one second switch (54a, 54b) comprises at least one control terminal (53a, 53b);
wherein the collector terminal galvanically connects to the at least one control terminal (53a, 53b); and
wherein the at least one second switch (54a, 54b) is configured to:
   read the processed logical signal from the at least one first switch (50) via the at least one control terminal (53a, 53b).

It is envisaged that the at least one control terminal (53a, 53b) comprises at least one gate terminal.

The present disclosure also pertains to any of the aforementioned components (3, 4, 7 - 9, 13, 27) involving at least one first switch (50), wherein the at least one switching circuit (29, 37 - 61) comprises the at least one first switch (50).

The instant disclosure also deals with any of the aforementioned components (3, 4, 7 - 9, 13, 27) involving at least one control terminal (53a, 53b), wherein the at least one second switch (54a, 54b) comprises a first field-effect transistor and a second field-effect transistor;
wherein the first field-effect transistor is separate from the second field-effect transistor;
wherein the first field-effect transistor comprises a first source terminal and a first gate terminal;
wherein the second field-effect transistor comprises a second source terminal and a second gate terminal;
wherein the first source terminal directly and electrically connects to the second source terminal;
wherein the first gate terminal directly and electrically connects to the second gate terminal; and
wherein the at least one control terminal (53a, 53b) comprises the first gate terminal and the second gate terminal.

The present disclosure also deals with any of the aforementioned components (3, 4, 7 - 9, 13, 27) involving at least one control terminal (53a, 53b), wherein the at least one second switch (54a, 54b) comprises a first metal-oxide semiconductor field-effect transistor and a second metal-oxide semiconductor field-effect transistor;
wherein the first metal-oxide semiconductor field-effect transistor is separate from the second metal-oxide semiconductor field-effect transistor;
wherein the first metal-oxide semiconductor field-effect transistor comprises a first source terminal and a first gate terminal;
wherein the second metal-oxide semiconductor field-effect transistor comprises a second source terminal and a second gate terminal;
wherein the first source terminal directly and electrically connects to the second source terminal;
wherein the first gate terminal directly and electrically connects to the second gate terminal; and
wherein the at least one control terminal (53a, 53b) comprises the first gate terminal and the second gate terminal.

The present disclosure still deals with any of the aforementioned components (3, 4, 7 - 9, 13, 27) involving at least one control terminal (53a, 53b), wherein the at least one second switch (54a, 54b) comprises a first enhancement mode N-channel metal-oxide semiconductor field-effect transistor and a second enhancement mode N-channel metal-oxide semiconductor field-effect transistor;
wherein the first enhancement mode N-channel metal-oxide semiconductor field-effect transistor is separate from the second enhancement mode N-channel metal-oxide semiconductor field-effect transistor;
wherein the first enhancement mode N-channel metal-oxide semiconductor field-effect transistor comprises a first source terminal and a first gate terminal;
wherein the second enhancement mode N-channel metal-oxide semiconductor field-effect transistor comprises a second source terminal and a second gate terminal;
wherein the first source terminal directly and electrically connects to the second source terminal;
wherein the first gate terminal directly and electrically connects to the second gate terminal; and
wherein the at least one control terminal (53a, 53b) comprises the first gate terminal and the second gate terminal.

A direct electrical connection means that no other electric components are arranged in between the two directly electrically connected components.

The arrangement comprising two field-effect transistors (53a, 53b, 54a, 54b) mitigates mechanical problems such as mechanical wear and contact bounce.

The present disclosure still deals with any of the aforementioned components (3, 4, 7 - 9, 13, 27) involving at least one second switch (54a, 54b), wherein the at least one second switch (54a, 54b) comprises at least one electric relay.

The present disclosure also deals with any of the aforementioned components (3, 4, 7 - 9, 13, 27) involving at least one second switch (54a, 54b), wherein the at least one second switch (54a, 54b) is at least one electric relay.

The arrangement comprising an electric relay mitigates capacitive disturbances and charge transfers caused by the gate electrodes (53a, 53b).

The present disclosure still deals with any of the aforementioned components (3, 4, 7 - 9, 13, 27), wherein the status signal comprises a binary signal indicative either of
- the open circuit condition or of
- the short circuit condition.

The instant disclosure also deals with any of the aforementioned components (3, 4, 7 - 9, 13, 27), wherein the status signal is a binary signal indicative either of
- the open circuit condition or of
- the short circuit condition.

It is envisaged that the status signal is an electric status signal. It is still envisaged that the status signal is a binary, electric signal. It is still envisaged that the status signal is an electronic status signal. It is still further envisaged that the status signal is a binary, electronic signal or a binary, electronic status signal.

The present disclosure also deals with any of the aforementioned components (3, 4, 7 - 9, 13, 27), wherein the at least one switching circuit (29, 37 - 61) is configured to electronically process the status signal.

The instant disclosure also deals with any of the aforementioned components (3, 4, 7 - 9, 13, 27), wherein the at least one switching circuit (29, 37 - 61) is configured to:
determine if the status signal indicates the open circuit condition or the short circuit condition.

The present disclosure still deals with any of the aforementioned components (3, 4, 7 - 9, 13, 27), wherein the at least one switching circuit (29, 37 - 61) is configured to:
process the status signal; and
determine if the status signal indicates the open circuit condition or the short circuit condition.

The instant disclosure still further deals with any of the aforementioned components (3, 4, 7 - 9, 13, 27), wherein the at least one switching circuit (29, 37 - 61) is configured to:
process the status signal to determine if the status signal indicates the open circuit condition or the short circuit condition.

The present disclosure still deals with any of the aforementioned components (3, 4, 7 - 9, 13, 27), wherein the at least one tangible, electric interface (30, 32, 35, 36) comprises a socket (29) arranged on the outer surface of the component (3, 4, 7 - 9, 13, 27), wherein the socket (29) is configured to:
receive a plug (28) and/or a bridge connection (35) such that the at least one tangible, electric interface (30, 32, 35, 36) is in the short circuit condition.

The instant disclosure still deals with any of the aforementioned components (3, 4, 7 - 9, 13, 27), wherein the at least one tangible, electric interface (30, 32, 35, 36) comprises a socket (29) arranged on the outer surface of the component (3, 4, 7 - 9, 13, 27), wherein the socket (29) is configured to:
receive a plug (28), the plug (28) having a bridge connection (35) and/or a jumper, such that the at least one tangible, electric interface (30, 32, 35, 36) is in the short circuit condition.

The configuration involving a socket (29) and a plug (28) and/or a bridge connection (35) alleviates issues due to an inadvertently operated electric switch (36). In other words, issues due to personnel error are mitigated.

The present disclosure still deals with any of the aforementioned components (3, 4, 7 - 9, 13, 27), wherein the at least one tangible, electric interface (30, 32, 35, 36) comprises at least one accessible, electromechanical switch (36);
wherein the at least one tangible, electric interface (30, 32, 35, 36) comprises an outer surface; and
wherein the at least one accessible, electromechanical switch (36) is arranged on the outer surface of the at least one tangible, electric interface (30, 32, 35, 36) such that the at least one accessible, electromechanical switch (36) is accessible to the user.

The instant disclosure also deals with any of the aforementioned components, wherein the at least one tangible, electric interface (30, 32, 35, 36) comprises at least one accessible, electromechanical switch (36);
wherein the at least one tangible, electric interface (30, 32, 35, 36) comprises an outer surface;
wherein the at least one accessible, electromechanical switch (36) is arranged on the outer surface of the at least one tangible, electric interface (30, 32, 35, 36) such that the at least one accessible, electromechanical switch (36) is accessible to the user;
wherein the at least one accessible, electromechanical switch (36) has a closed position such that the at least one tangible, electric interface (30, 32, 35, 36) is in the short circuit condition; and
wherein the at least one accessible, electromechanical switch (36) has an open position such that the at least one tangible, electric interface (30, 32, 35, 36) is in the open circuit condition.

The instant disclosure also deals with any of the aforementioned components (3, 4, 7 - 9, 13, 27), wherein the component (3, 4, 7 - 9, 13, 27) comprises at least one of:
- a fan (3),
- an actuator of the fan (3),
- a damper (4),
- an actuator of the damper (4),
- a valve (7 - 9),
- an actuator of one of the valves (7 - 9),
- a flow sensor (13),
- a mass flow sensor (13),
- a flue gas sensor (27).

The instant disclosure still deals with any of the aforementioned components (3, 4, 7 - 9, 13, 27), wherein the component (3, 4, 7 - 9, 13, 27) is selected from one of:
- a fan (3),
- an actuator of the fan (3),
- a damper (4),
- an actuator of the damper (4),
- a valve (7 - 9),
- an actuator of one of the valves (7 - 9),
- a flow sensor (13),
- a mass flow sensor (13),
- a flue gas sensor (27).

A component (3, 4, 7 - 9, 13, 27) providing a bus termination allows bus terminations to be disabled or to be enabled by default, thereby reducing risks of on-site misconfiguration. More specifically, bus terminations can be configured prior to installation and/or prior to commissioning of a combustion appliance.

The present disclosure also deals with any of the aforementioned components (3, 4, 7 - 9, 13, 27), wherein the at least one switching circuit (29, 37 - 61) comprises a controller having a non-volatile memory, wherein the at least one switching circuit (29, 37 - 61) is configured to:
if the status signal indicates the open circuit condition:
use the controller of the at least one switching circuit (29, 37 - 61) to assign a first network address to the at least one component (3, 4, 7 - 9, 13, 27);
use the controller of the at least one switching circuit (29, 37 - 61) to store the first network address in the non-volatile memory;
if the status signal indicates the short circuit condition:
   use the controller of the at least one switching circuit (29, 37 - 61) to assign a second network address to the at least one component (3, 4, 7 - 9, 13, 27), the second network address being different from the first network address; and
   use the controller of the at least one switching circuit (29, 37 - 61) to store the second network address in the non-volatile memory.

The present disclosure still deals with any of the aforementioned components (3, 4, 7 - 9, 13, 27), wherein the component (3, 4, 7 - 9, 13, 27) comprises at least one controller, wherein the at least one controller of the component (3, 4, 7 - 9, 13, 27) communicatively connects to the at least one switching circuit (29, 37 - 61), wherein the at least one controller of the component (3, 4, 7 - 9, 13, 27) is configured to:
read the logical output signal from the at least one NOT gate;
process the logical output signal; and
if the logical output signal read from the at least one NOT gate indicates a logical LOW signal:
   assign a first bus address to the component (3, 4, 7 - 9, 13, 27);
   if the logical output signal read from the at least one NOT gate indicates a logical HIGH signal:
      assign a second bus address to the component (3, 4, 7 - 9, 13, 27), wherein the second bus address differs from the first bus address.

The instant disclosure still further deals with any of the aforementioned combustion appliances involving at least one controller of the component (3, 4, 7 - 9, 13, 27), wherein the least one controller of the component (3, 4, 7 - 9, 13, 27) is configured to:
use the assigned first network address or the assigned second network address to communicate with the controller (16) of the combustion appliance via the at least one first bus wire and via the at least one second bus wire.

The least one controller of the component (3, 4, 7 - 9, 13, 27) advantageously communicatively connects to the controller (16) of the combustion appliance.

The instant disclosure also deals with a combustion appliance comprising a controller (16), a burner (1) and a heat consumer (2) in operative communication with the burner (1), the combustion appliance also comprising at least one component (3, 4, 7 - 9, 13, 27) as described herein;
wherein the at least one component (3, 4, 7 - 9, 13, 27) communicatively connects to the controller (16) via the at least one first bus wire and via the at least one second bus wire.

The present disclosure still deals with any of the aforementioned combustion appliances, wherein the controller (16) of the combustion appliance is configured to:
assign a unique identifier in the form of a network address to the at least one component (3, 4, 7 - 9, 13, 27);
send the network address to the address to the at least one component (3, 4, 7 - 9, 13, 27); and
wherein the at least one component (3, 4, 7 - 9, 13, 27) is configured to:
   receive the network address.

It is envisaged that the wherein the at least one component (3, 4, 7 - 9, 13, 27) comprises a memory such as a non-volatile memory and is configured to:
receive the network address;
store the network address in its memory; and
use the network address for future communications with the controller (16) of the combustion appliance.

The network address can comprise a logical network address. The network address can also be a logical network address.

The instant disclosure still deals with any of the aforementioned combustion appliances, wherein the controller (16) of the combustion appliance is configured to:
read the status signal from the at least one tangible, electric interface (30, 32, 35, 36);
process the status signal; and
if the status signal indicates the open circuit condition:
   assign a first address to the at least one component (3, 4, 7 - 9, 13, 27);
   if the status signal indicates the short circuit condition:
      assign a second address to the at least one component (3, 4, 7 - 9, 13, 27), the second address being different from the first address.

The present disclosure still deals with any of the aforementioned combustion appliances involving a controller of the at least one switching circuit (29, 37 - 61), wherein the controller of the at least one switching circuit (29, 37 - 61) is configured to:
use the assigned first network address or the assigned second network address to communicate with the controller (16) of the combustion appliance via the at least one first bus wire and via the at least one second bus wire.

It is envisaged that the first address comprises a first bus address and that the second address comprises a second bus address. It is still envisaged that the first address is a first bus address and that the second address is a second bus address. It is also envisaged that the first address comprises a first address of a bus and that the second address comprises a second address of the bus. It is still envisaged that the first address is a first address of a bus and that the second address is a second address of the bus.

By mapping an address of the bus to the component (3, 4, 7 - 9, 13, 27), the component (3, 4, 7 - 9, 13, 27) can readily be identified. This identification of the component (3, 4, 7 - 9, 13, 27) depends on the termination of the bus.

Any steps of a procedure according to the present disclosure can be embodied in hardware and/or in a software module executed by a processor. Any steps of such a procedure can also be embodied in a software module executed by a processor inside a container using operating system level virtualisation. Any steps of such a procedure can still be embodied in a cloud computing arrangement. It is envisaged that any steps of a procedure according to the present disclosure is implemented in a combination of the above embodiments. The software may include a firmware and/or a hardware driver run by the operating system and/or an application program. Thus, the disclosure also relates to a non-transitory computer program product for performing the operations presented herein. If implemented in software, the functions described may be stored as one or more instructions on a computer-readable and non-transitory medium. Storage media that can be used include, by way of non-limiting examples, random access memory (RAM) and/or read only memory (ROM) and/or flash memory. Storage media can, by way of non-limiting examples, also include EPROM memory and/or EEPROM memory and/or registers and/or a hard disk and/or a removable disk. Further storage media can, by way of non-limiting examples, include other optical disks and/or any available media that can be accessed by a computer. Storage media can still, by way of non-limiting example, include any other IT equipment and appliance.

It should be understood that the foregoing relates only to certain embodiments of the invention.

Numerous changes may be made therein without departing from the scope of the invention as defined by the following claims. It should also be understood that the disclosure is not restricted to the illustrated embodiments.

### Reference numerals

1 burner
2 heat consumer (heat exchanger), in particular combustion chamber
3 fan
4 damper or valve (with motorized adjustment)
5 fluid flow (mass flow) in main duct, air flow, air throughflow
6 fluid flow of a combustible fluid, fuel throughput
7, 8 safety valve
9 damper or valve (with motorized adjustment)
10 waste gas flow, exhaust gas flow
11 feed duct (air duct)
12 connector
13 flow sensor
14 flow resistance element (diaphragm)
15 throughflow (flow, mass flow) in the side duct
16 controller
17 - 22 signal lines
23 air inlet
24 side duct
25 exhaust gas path
26 signal line
27 flue gas sensor
28 plug
29 socket
30 - 34 pins
35 bridge connection
36 switch
37 - 41 connectors
42 terminal
43 resistor
44 switch
45 resistor
46 capacitor
47 resistor
48a - 48c terminals
49 terminal
50 switch
51, 52 resistors
53a, 53b control terminal
54a, 54b switch
55, 56 resistors
57 terminal
58 capacitor
59 impedance
60, 61 terminals

## Claims

1. A component (3, 4, 7 - 9, 13, 27) for a combustion appliance, the component (3, 4, 7 - 9, 13, 27) comprising an outer surface and at least one tangible, electric interface (30, 32, 35, 36) configured to be electrically connected by a user such that the at least one tangible, electric interface (30, 32, 35, 36) is in a short circuit condition and configured to be electrically disconnected by the user such that the at least one tangible, electric interface (30, 32, 35, 36) is in an open circuit condition;
wherein the at least one tangible, electric interface (30, 32, 35, 36) is arranged on the outer surface of the component (3, 4, 7 - 9, 13, 27) such that the at least one tangible, electric interface (30, 32, 35, 36) is accessible to the user;
the component (3, 4, 7 - 9, 13, 27) also comprising at least one first bus wire and at least one second bus wire, wherein the at least one first bus wire is different from the at least one second bus wire;
the component (3, 4, 7 - 9, 13, 27) also comprising at least one switching circuit (29, 37 - 61), wherein the at least one switching circuit (29, 37 - 61) comprises at least one switchable electric impedance (54a, 54b, 59);
wherein the at least one switchable electric impedance (54a, 54b, 59) electrically connects to the at least one first bus wire;
wherein the at least one switching circuit (29, 37 - 61) is configured to:
read from the at least one tangible, electric interface (30, 32, 35, 36) a status signal indicative of the open circuit condition or of the short circuit condition;
process the status signal; and
if the status signal indicates the open circuit condition:
either activate the at least one switchable electric impedance (54a, 54b, 59) such that the at least one switchable electric impedance (54a, 54b, 59) electrically connects to the at least one second bus wire, thereby electrically connecting the at least one first bus wire to the at least one second bus wire via the at least one switchable electric impedance (54a, 54b, 59), or
deactivate the at least one switchable electric impedance (54a, 54b, 59) such that the at least one switchable electric impedance (54a, 54b, 59) electrically disconnects from the at least one second bus wire, thereby electrically disconnecting the at least one first bus wire from the at least one second bus wire.

2. The component (3, 4, 7 - 9, 13, 27) according to claim 1, wherein the at least one switching circuit (29, 37 - 61) is configured to:
if the status signal indicates the short circuit condition:
deactivate the at least one switchable electric impedance (54a, 54b, 59) such that the at least one switchable electric impedance (54a, 54b, 59) electrically disconnects from the at least one second bus wire, thereby electrically disconnecting the at least one first bus wire from the at least one second bus wire.

3. The component (3, 4, 7 - 9, 13, 27) according to any of the claims 1 to 2, wherein the at least one switching circuit (29, 37 - 61) comprises at least one NOT gate (42 - 49) electrically connected to the at least one tangible, electric interface (30, 32, 35, 36), wherein the at least one NOT gate (42 - 49) is configured to:
read from the at least one tangible, electric interface (30, 32, 35, 36) a status signal indicative of the open circuit condition or of the short circuit condition;
process the status signal; and
if the status signal indicates the open circuit condition:
generate a logical LOW output signal.

4. The component (3, 4, 7 - 9, 13, 27) according to claim 3, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and to the at least one electric resistor (59), wherein the at least one first switch (50) is configured to:
read the logical LOW output signal from the at least one NOT gate;
process the logical LOW signal;
transmit the processed logical signal to the at least one second switch (54a, 54b);
wherein the at least one second switch (54a, 54b) is configured to:
read the processed logical signal from the at least one first switch (50); and
electrically connect the at least one electric resistor (59) to the at least one second bus wire.

5. The component (3, 4, 7 - 9, 13, 27) according to any of the claims 1 to 2, wherein the at least one switching circuit (29, 37 - 61) comprises at least one NOT gate (42 - 49) electrically connected to the at least one tangible, electric interface (30, 32, 35, 36), wherein the at least one NOT gate (42 - 49) is configured to:
read from the at least one tangible, electric interface (30, 32, 35, 36) a status signal indicative of the open circuit condition or of the short circuit condition;
process the status signal; and
if the status signal indicates the short circuit condition:
generate a logical HIGH output signal.

6. The component (3, 4, 7 - 9, 13, 27) according to claim 5, wherein the component (3, 4, 7 - 9, 13, 27) comprises at least one first switch (50) electrically connected to the at least one NOT gate and wherein the at least one switchable electric impedance (54a, 54b, 59) comprises at least one second switch (54a, 54b) and at least one electric resistor (59), wherein the at least one second switch (54a, 54b) electrically connects to the at least one first switch (50) and to the at least one electric resistor (59), wherein the at least one first switch (50) is configured to:
read the logical HIGH output signal from the at least one NOT gate;
process the logical HIGH signal;
transmit the processed logical signal to the at least one second switch (54a, 54b);
wherein the at least one second switch (54a, 54b) is configured to:
read the processed logical signal from the at least one first switch (50); and
electrically disconnect the at least one electric resistor (59) from the at least one second bus wire.

7. The component (3, 4, 7 - 9, 13, 27) according to any of the claims 4 or 6, wherein the at least one second switch (54a, 54b) comprises at least one control terminal (53a, 53b), wherein the at least one second switch (54a, 54b) is configured to:
read the processed logical signal from the at least one first switch (50) via the at least one control terminal (53a, 53b).

8. The component (3, 4, 7 - 9, 13, 27) according to claim 7, wherein the at least one second switch (54a, 54b) comprises a first field-effect transistor and a second field-effect transistor;
wherein the first field-effect transistor is separate from the second field-effect transistor;
wherein the first field-effect transistor comprises a first source terminal and a first gate terminal;
wherein the second field-effect transistor comprises a second source terminal and a second gate terminal;
wherein the first source terminal directly and electrically connects to the second source terminal;
wherein the first gate terminal directly and electrically connects to the second gate terminal; and
wherein the at least one control terminal (53a, 53b) comprises the first gate terminal and the second gate terminal.

9. The component (3, 4, 7 - 9, 13, 27) according to any of the claims 4 or 6 or 7, wherein the at least one second switch (54a, 54b) comprises at least one electric relay.

10. The component (3, 4, 7 - 9, 13, 27) according to any of the claims 1 to 9, wherein the status signal comprises a binary signal indicative either of
- the open circuit condition or of
- the short circuit condition.

11. The component (3, 4, 7 - 9, 13, 27) according to any of the claims 1 to 10, wherein the at least one switching circuit (29, 37 - 61) is configured to electronically process the status signal.

12. The component (3, 4, 7 - 9, 13, 27) according to any of the claims 1 to 11, wherein the at least one tangible, electric interface (30, 32, 35, 36) comprises a socket (29) arranged on the outer surface of the component (3, 4, 7 - 9, 13, 27), wherein the socket (29) is configured to:
receive a plug (28) and/or a bridge connection (35) such that the at least one tangible, electric interface (30, 32, 35, 36) is in the short circuit condition.

13. The component (3, 4, 7 - 9, 13, 27) according to any of the claims 1 to 12, wherein the component (3, 4, 7 - 9, 13, 27) comprises at least one of:
- a fan (3),
- an actuator of the fan (3),
- a damper (4),
- an actuator of the damper (4),
- a valve (7 - 9),
- an actuator of one of the valves (7 - 9),
- a flow sensor (13),
- a mass flow sensor (13),
- a flue gas sensor (27).

14. The component (3, 4, 7 - 9, 13, 27) according to any of the claims 3 to 9, wherein the component (3, 4, 7 - 9, 13, 27) comprises at least one controller, wherein the at least one controller of the component (3, 4, 7 - 9, 13, 27) communicatively connects to the at least one switching circuit (29, 37 - 61), wherein the at least one controller of the component (3, 4, 7 - 9, 13, 27) is configured to:
read the logical output signal from the at least one NOT gate;
process the logical output signal; and
if the logical output signal read from the at least one NOT gate indicates a logical LOW signal:
assign a first bus address to the component (3, 4, 7 - 9, 13, 27);
if the logical output signal read from the at least one NOT gate indicates a logical HIGH signal:
assign a second bus address to the component (3, 4, 7 - 9, 13, 27), wherein the second bus address differs from the first bus address.

15. A combustion appliance comprising a controller (16), a burner (1) and a heat consumer (2) in operative communication with the burner (1), the combustion appliance also comprising at least one component (3, 4, 7 - 9, 13, 27) according to claim 14;
wherein the at least one component (3, 4, 7 - 9, 13, 27) communicatively connects to the controller (16) of the combustion appliance via the at least one first bus wire and via the at least one second bus wire.

## Patentansprüche

1. Komponente (3, 4, 7 - 9, 13, 27) für eine Verbrennungsvorrichtung, wobei die Komponente (3, 4, 7 - 9, 13, 27) eine Außenfläche und mindestens eine greifbare elektrische Schnittstelle (30, 32, 35, 36) umfasst, die so konfiguriert ist, dass sie von einem Benutzer elektrisch so angeschlossen werden kann, dass sie sich in einem Kurzschlusszustand befindet, und von dem Benutzer elektrisch getrennt werden kann, so dass sie sich in einem Zustand mit offenem Stromkreis befindet,
wobei die mindestens eine greifbare elektrische Schnittstelle (30, 32, 35, 36) so an der Außenfläche der Komponente (3, 4,7 - 9, 13, 27) angeordnet ist, dass sie für den Benutzer zugänglich ist;
wobei die Komponente (3, 4, 7 - 9, 13, 27) auch mindestens einen ersten und mindestens einen zweiten Busdraht umfasst, wobei sich der mindestens eine erste Busdraht von dem mindestens einen zweiten Busdraht unterscheidet;
wobei die Komponente (3, 4, 7 - 9, 13, 27) auch mindestens einen Schaltkreis (29, 37 - 61) umfasst, wobei der mindestens eine Schaltkreis (29, 37 - 61) mindestens eine schaltbare elektrische Impedanz (54a, 54b, 59) umfasst;
wobei sich die mindestens eine schaltbare elektrische Impedanz (54a, 54b, 59) elektrisch an den mindestens einen ersten Busdraht anschließen lässt;
wobei der mindestens eine Schaltkreis (29, 37 - 61) so konfiguriert ist, dass er:
an der mindestens einen greifbaren elektrischen Schnittstelle (30, 32, 35, 36) ein Zustandssignal ausliest, das den Zustand mit offenem Stromkreis oder den Kurzschlusszustand angibt;
das Zustandssignal verarbeitet; und,
wenn das Zustandssignal den Zustand mit offenem Stromkreis angibt:
entweder die mindestens eine schaltbare elektrische Impedanz (54a, 54b, 59) aktiviert, so dass diese elektrisch an den mindestens einen zweiten Busdraht angeschlossen wird, wodurch der mindestens eine erste Busdraht über die mindestens eine schaltbare elektrische Impedanz (54a, 54b, 59) elektrisch mit dem mindestens einen zweiten Busdraht verbunden wird, oder
die mindestens eine schaltbare elektrische Impedanz (54a, 59, 54b) deaktiviert, so dass diese elektrisch von dem mindestens einen zweiten Busdraht getrennt wird, wodurch der mindestens eine erste Busdraht elektrisch von dem mindestens einen zweiten Busdraht getrennt wird.

2. Die Komponente (3, 4, 7 - 9, 13, 27) nach Anspruch 1, wobei der mindestens eine Schaltkreis (29, 37 - 61) so konfiguriert ist, dass er,
wenn das Zustandssignal den Kurzschlusszustand angibt:
die mindestens eine schaltbare elektrische Impedanz (54a, 59, 54b) deaktiviert, so dass diese elektrisch von dem mindestens einen zweiten Busdraht getrennt wird, wodurch der mindestens eine erste Busdraht elektrisch von dem mindestens einen zweiten Busdraht getrennt wird.

3. Die Komponente (3, 4, 7 - 9, 13, 27) nach einem der Ansprüche 1 bis 2, wobei der mindestens eine Schaltkreis (29, 37 - 61) mindestens ein NICHT-Gatter (42 - 49) umfasst, das elektrisch an die mindestens eine greifbare elektrische Schnittstelle (30, 32, 35, 36) angeschlossen ist, wobei das mindestens eine NICHT-Gatter (42 - 49) so konfiguriert ist, dass es:
an der mindestens einen greifbaren elektrischen Schnittstelle (30, 32, 35, 36) ein Zustandssignal ausliest, das den Zustand mit offenem Stromkreis oder den Kurzschlusszustand angibt,
das Zustandssignal verarbeitet; und,
wenn das Zustandssignal den Zustand mit offenem Stromkreis angibt:
ein Logisch-LOW-Ausgangssignal generiert.

4. Die Komponente (3, 4, 7 - 9, 13, 27) nach Anspruch 3, wobei die Komponente (3, 4, 7 - 9, 13, 27) mindestens einen ersten Schalter (50) umfasst, der elektrisch an das mindestens eine NICHT-Gatter angeschlossen ist, und die mindestens eine schaltbare elektrische Impedanz (54a, 54b, 59) mindestens einen zweiten Schalter (54a, 54b) und mindestens einen elektrischen Widerstand (59) umfasst, wobei der mindestens eine zweite Schalter (54a, 54b) elektrisch an den mindestens einen ersten Schalter (50) und den mindestens einen elektrischen Widerstand (59) angeschlossen ist, wobei der mindestens eine erste Schalter (50) so konfiguriert ist, dass er:
das Logisch-Low-Ausgangssignal aus dem mindestens einen NICHT-Gatter ausliest,
das Logisch-LOW-Signal verarbeitet,
das verarbeitete logische Signal zu dem mindestens einen zweiten Schalter (54a, 54b) weiterleitet,
wobei der mindestens eine zweite Schalter (54a, 54b) so konfiguriert ist, dass er:
das verarbeitete logische Signal aus dem mindestens einen ersten Schalter (50) ausliest und
den mindestens einen elektrischen Widerstand (59) elektrisch an den mindestens einen zweiten Busdraht anschließt.

5. Die Komponente (3, 4, 7 - 9, 13, 27) nach einem der Ansprüche 1 bis 2, wobei der mindestens eine Schaltkreis (29, 37 - 61) mindestens ein NICHT-Gatter (42 - 49) umfasst, das elektrisch an die mindestens eine greifbare elektrische Schnittstelle (30, 32, 35, 36) angeschlossen ist, wobei das mindestens eine NICHT-Gatter (42 - 49) so konfiguriert ist, dass es:
an der mindestens einen greifbaren elektrischen Schnittstelle (30, 32, 35, 36) ein Zustandssignal ausliest, das den Zustand mit offenem Stromkreis oder den Kurzschlusszustand angibt,
das Zustandssignal verarbeitet und,
wenn das Zustandssignal den Kurzschlusszustand angibt:
ein Logisch-HIGH-Ausgangssignal generiert.

6. Die Komponente (3, 4, 7 - 9, 13, 27) nach Anspruch 5, wobei die Komponente (3, 4, 7 - 9, 13, 27) mindestens einen ersten Schalter (50) umfasst, der elektrisch an das mindestens eine NICHT-Gatter angeschlossen ist, und die mindestens eine schaltbare elektrische Impedanz (54a, 54b, 59) mindestens einen zweiten Schalter (54a, 54b) und mindestens einen elektrischen Widerstand (59) umfasst, wobei der mindestens eine zweite Schalter (54a, 54b) elektrisch an den mindestens einen ersten Schalter (50) und den mindestens einen elektrischen Widerstand (59) angeschlossen ist, wobei der mindestens eine erste Schalter (50) so konfiguriert ist, dass er:
das Logisch-HIGH-Ausgangssignal aus dem mindestens einen NICHT-Gatter ausliest,
das Logisch-HIGH-Signal verarbeitet,
das verarbeitete logische Signal zu dem mindestens einen zweiten Schalter (54a, 54b) weiterleitet,
wobei der mindestens eine zweite Schalter (54a, 54b) so konfiguriert ist, dass er:
das verarbeitete logische Signal aus dem mindestens einen ersten Schalter (50) ausliest und
den mindestens einen elektrischen Widerstand (59) elektrisch von dem mindestens einen zweiten Busdraht trennt.

7. Die Komponente (3, 4, 7 - 9, 13, 27) nach Anspruch 4 oder 6, wobei der mindestens eine zweite Schalter (54a, 54b) mindestens einen Steueranschluss (53a, 53b) umfasst, wobei der mindestens eine zweite Schalter (54a, 54b) so konfiguriert ist, dass er:
das verarbeitete logische Signal über den mindestens einen Steueranschluss (53a, 53b) aus dem mindestens einen ersten Schalter (50) ausliest.

8. Die Komponente (3, 4, 7 - 9, 13, 27) nach Anspruch 7, wobei der mindestens eine zweite Schalter (54a, 54b) einen ersten und einen zweiten Feldeffekttransistor umfasst,
wobei der erste Feldeffekttransistor von dem zweiten Feldeffekttransistor getrennt ist,
wobei der erste Feldeffekttransistor einen ersten Source-Anschluss und einen ersten Gate-Anschluss umfasst,
wobei der zweite Feldeffekttransistor einen zweiten Source-Anschluss und einen zweiten Gate-Anschluss umfasst,
wobei der erste Source-Anschluss elektrisch direkt an den zweiten Source-Anschluss angeschlossen ist,
wobei der erste Gate-Anschluss elektrisch direkt an den zweiten Gate-Anschluss angeschlossen ist und
wobei der mindestens eine Steueranschluss (53a, 53b) den ersten und den zweiten Gate-Anschluss umfasst.

9. Die Komponente (3, 4, 7 - 9, 13, 27) nach Anspruch 4 oder 6 oder 7, wobei der mindestens eine zweite Schalter (54a, 54b) mindestens ein elektrisches Relais umfasst.

10. Die Komponente (3, 4, 7 - 9, 13, 27) nach einem der Ansprüche 1 bis 9, wobei das Zustandssignal ein binäres Signal umfasst, das entweder
- den Zustand mit offenem Stromkreis oder
- den Kurzschlusszustand angibt.

11. Die Komponente (3, 4, 7 - 9, 13, 27) nach einem der Ansprüche 1 bis 10, wobei der mindestens eine Schaltkreis (29, 37 - 61) so konfiguriert ist, dass er das Zustandssignal elektronisch verarbeitet.

12. Die Komponente (3, 4, 7 - 9, 13, 27) nach einem der Ansprüche 1 bis 11, wobei die mindestens eine greifbare elektrische Schnittstelle (30, 32, 35, 36) eine Anschlussdose (29) umfasst, die an der Außenfläche der Komponente (3, 4, 7 - 9, 13, 27) angeordnet ist, wobei die Anschlussdose (29) so konfiguriert ist, dass sie:
einen Stecker (28) und/oder eine Brückenschaltung (35) so aufnimmt, dass sich die mindestens eine greifbare elektrische Schnittstelle (30, 32, 35, 36) im Kurzschlusszustand befindet.

13. Die Komponente (3, 4, 7 - 9, 13, 27) nach einem der Ansprüche 1 bis 12, wobei die Komponente (3, 4, 7 - 9, 13, 27) mindestens eines der folgenden Elemente umfasst:
- ein Gebläse (3),
- ein Stellelement für das Gebläse (3),
- eine Klappe (4),
- ein Stellelement für die Klappe (4),
- ein Ventil (7 - 9),
- ein Stellelement für eines der Ventile (7 - 9),
- einen Durchflusssensor (13),
- einen Mengendurchflusssensor (13),
- einen Abgassensor (27).

14. Die Komponente (3, 4, 7 - 9, 13, 27) nach einem der Ansprüche 3 bis 9, wobei die Komponente (3, 4, 7 - 9, 13, 27) mindestens eine Steuerung umfasst, wobei die mindestens eine Steuerung der Komponente (3, 4, 7 - 9, 13, 27) mit dem mindesten einen Schaltkreis (29, 37 - 61) kommunikativ verbunden ist, wobei die mindestens eine Steuerung der Komponente (3, 4, 7 - 9, 13, 27) so konfiguriert ist, dass sie:
das logische Ausgangssignal aus dem mindestens einen NICHT-Gatter ausliest,
das logische Ausgangssignal verarbeitet und,
wenn das aus dem mindestens einen NICHT-Gatter ausgelesene logische Ausgangssignal ein Logisch-LOW-Signal angibt:
der Komponente (3, 4, 7 - 9, 13, 27) eine erste Busadresse zuweist,
wenn das aus dem mindestens einen NICHT-Gatter ausgelesene logische Ausgangssignal ein Logisch-HIGH-Signal angibt:
der Komponente (3, 4, 7 - 9, 13, 27) eine zweite Busadresse zuweist, wobei sich die zweite Busadresse von der ersten Busadresse unterscheidet.

15. Verbrennungsvorrichtung mit einer Steuerung (16), einem Brenner (1) und einem mit dem Brenner (1) in Betriebsverbindung stehenden Wärmeverbraucher (2), wobei die Verbrennungsvorrichtung auch mindestens eine Komponente (3, 4, 7 - 9, 13, 27) nach Anspruch 14 umfasst;
wobei die mindestens eine Komponente (3, 4, 7 - 9, 13, 27) über den mindestens einen ersten und den mindestens einen zweiten Busdraht mit der Steuerung (16) der Verbrennungsvorrichtung kommunikativ verbunden ist.

## Revendications

1. Composant (3, 4, 7-9, 13, 27) pour un appareil à combustion, le composant (3, 4, 7-9, 13, 27) comprenant une surface externe et au moins une interface électrique tangible (30, 32, 35, 36) configurée pour être connectée électriquement par un utilisateur de telle sorte que l'au moins une interface électrique tangible (30, 32, 35, 36) soit dans une condition de court-circuit et configurée pour être déconnectée électriquement par l'utilisateur de telle sorte que l'au moins une interface électrique tangible (30, 32, 35, 36) soit dans une condition de circuit ouvert ;
dans lequel l'au moins une interface électrique tangible (30, 32, 35, 36) est disposée sur la surface externe du composant (3, 4, 7-9, 13, 27) de telle sorte que l'au moins une interface électrique tangible (30, 32, 35, 36) soit accessible à l'utilisateur ;
le composant (3, 4, 7-9, 13, 27) comprenant également au moins un premier fil de bus et au moins un deuxième fil de bus, dans lequel l'au moins un premier fil de bus est différent de l'au moins un deuxième fil de bus ;
le composant (3, 4, 7-9, 13, 27) comprenant également au moins un circuit de commutation (29, 37-61), dans lequel l'au moins un circuit de commutation (29, 37-61) comprend au moins une impédance électrique commutable (54a, 54b, 59) ;
dans lequel l'au moins une impédance électrique commutable (54a, 54b, 59) se connecte électriquement à l'au moins un premier fil de bus ;
dans lequel l'au moins un circuit de commutation (29, 37-61) est configuré pour :
lire à partir de l'au moins une interface électrique tangible (30, 32, 35, 36) un signal d'état indiquant la condition de circuit ouvert ou la condition de court-circuit ;
traiter le signal d'état ; et
si le signal d'état indique la condition de circuit ouvert :
soit activer l'au moins une impédance électrique commutable (54a, 54b, 59) de telle sorte que l'au moins une impédance électrique commutable (54a, 54b, 59) se connecte électriquement à l'au moins un deuxième fil de bus, connectant ainsi électriquement l'au moins un premier fil de bus à l'au moins un deuxième fil de bus via l'au moins une impédance électrique commutable (54a, 54b, 59),
soit désactiver l'au moins une impédance électrique commutable (54a, 54b, 59) de telle sorte que l'au moins une impédance électrique commutable (54a, 54b, 59) se déconnecte électriquement de l'au moins un deuxième fil de bus, déconnectant ainsi électriquement l'au moins un premier fil de bus de l'au moins un deuxième fil de bus.

2. Le composant (3, 4, 7-9, 13, 27) selon la revendication 1, dans lequel l'au moins un circuit de commutation (29, 37-61) est configuré pour :
si le signal d'état indique la condition de court-circuit :
désactiver l'au moins une impédance électrique commutable (54a, 54b, 59) de telle sorte que l'au moins une impédance électrique commutable (54a, 54b, 59) se déconnecte électriquement de l'au moins un deuxième fil de bus, déconnectant ainsi électriquement l'au moins un premier fil de bus de l'au moins un deuxième fil de bus.

3. Le composant (3, 4, 7-9, 13, 27) selon l'une des revendications 1 à 2, dans lequel l'au moins un circuit de commutation (29, 37-61) comprend au moins une porte NOT (42-49) connectée électriquement à l'au moins une interface électrique tangible (30, 32, 35, 36), dans lequel l'au moins une porte NOT (42-49) est configurée pour :
lire à partir de l'au moins une interface électrique tangible (30, 32, 35, 36) un signal d'état indiquant la condition de circuit ouvert ou la condition de court-circuit ;
traiter le signal d'état ; et
si le signal d'état indique la condition de circuit ouvert :
générer un signal de sortie logique LOW.

4. Le composant (3, 4, 7-9, 13, 27) selon la revendication 3, dans lequel le composant (3, 4, 7-9, 13, 27) comprend au moins un premier commutateur (50) connecté électriquement à l'au moins une porte NOT et dans lequel l'au moins une impédance électrique commutable (54a, 54b, 59) comprend au moins un deuxième commutateur (54a, 54b) et au moins une résistance électrique (59), dans lequel l'au moins un deuxième commutateur (54a, 54b) se connecte électriquement à l'au moins un premier commutateur (50) et à l'au moins une résistance électrique (59), dans lequel l'au moins un premier commutateur (50) est configuré pour :
lire le signal de sortie logique LOW à partir de l'au moins une porte NOT ;
traiter le signal logique LOW ;
transmettre le signal logique traité à l'au moins un deuxième commutateur (54a, 54b) ;
dans lequel l'au moins un deuxième commutateur (54a, 54b) est configuré pour :
lire le signal logique traité à partir de l'au moins un premier commutateur (50) ; et
connecter électriquement l'au moins une résistance électrique (59) à l'au moins un deuxième fil de bus.

5. Le composant (3, 4, 7-9, 13, 27) selon l'une des revendications 1 à 2, dans lequel l'au moins un circuit de commutation (29, 37-61) comprend au moins une porte NOT (42-49) connectée électriquement à l'au moins une interface électrique tangible (30, 32, 35, 36), dans lequel l'au moins une porte NOT (42-49) est configurée pour :
lire à partir de l'au moins une interface électrique tangible (30, 32, 35, 36) un signal d'état indiquant la condition de circuit ouvert ou la condition de court-circuit ;
traiter le signal d'état ; et
si le signal d'état indique la condition de court-circuit :
générer un signal de sortie logique HIGH.

6. Le composant (3, 4, 7-9, 13, 27) selon la revendication 5, dans lequel le composant (3, 4, 7-9, 13, 27) comprend au moins un premier commutateur (50) connecté électriquement à l'au moins une porte NOT et dans lequel l'au moins une impédance électrique commutable (54a, 54b, 59) comprend au moins un deuxième commutateur (54a, 54b) et au moins une résistance électrique (59), dans lequel l'au moins un deuxième commutateur (54a, 54b) se connecte électriquement à l'au moins un premier commutateur (50) et à l'au moins une résistance électrique (59), dans lequel l'au moins un premier commutateur (50) est configuré pour :
lire le signal de sortie logique HIGH à partir de l'au moins une porte NOT ;
traiter le signal logique HIGH ;
transmettre le signal logique traité à l'au moins un deuxième commutateur (54a, 54b) ;
dans lequel l'au moins un deuxième commutateur (54a, 54b) est configuré pour :
lire le signal logique traité à partir de l'au moins un premier commutateur (50) ; et
déconnecter électriquement l'au moins une résistance électrique (59) de l'au moins un deuxième fil de bus.

7. Le composant (3, 4, 7-9, 13, 27) selon l'une des revendications 4 ou 6, dans lequel l'au moins un deuxième commutateur (54a, 54b) comprend au moins une borne de commande (53a, 53b), dans lequel l'au moins un deuxième commutateur (54a, 54b) est configuré pour :
lire le signal logique traité à partir de l'au moins un premier commutateur (50) via l'au moins une borne de commande (53a, 53b).

8. Le composant (3, 4, 7-9, 13, 27) selon la revendication 7, dans lequel l'au moins un deuxième commutateur (54a, 54b) comprend un premier transistor à effet de champ et un deuxième transistor à effet de champ ;
dans lequel le premier transistor à effet de champ est distinct du deuxième transistor à effet de champ ;
dans lequel le premier transistor à effet de champ comprend une première borne de source et une première borne de grille ;
dans lequel le deuxième transistor à effet de champ comprend une deuxième borne de source et une deuxième borne de grille ;
dans lequel la première borne de source se connecte directement et électriquement à la deuxième borne de source ;
dans lequel la première borne de grille se connecte directement et électriquement à la deuxième borne de grille ; et
dans lequel l'au moins une borne de commande (53a, 53b) comprend la première borne de grille et la deuxième borne de grille.

9. Le composant (3, 4, 7-9, 13, 27) selon l'une des revendications 4 ou 6 ou 7, dans lequel l'au moins un deuxième commutateur (54a, 54b) comprend au moins un relais électrique.

10. Le composant (3, 4, 7-9, 13, 27) selon l'une des revendications 1 à 9, dans lequel le signal d'état comprend un signal binaire indiquant soit
- la condition de circuit ouvert, ou
- la condition de court-circuit.

11. Le composant (3, 4, 7-9, 13, 27) selon l'une des revendications 1 à 10, dans lequel l'au moins un circuit de commutation (29, 37-61) est configuré pour traiter électroniquement le signal d'état.

12. Le composant (3, 4, 7-9, 13, 27) selon l'une des revendications 1 à 11, dans lequel l'au moins une interface électrique tangible (30, 32, 35, 36) comprend une prise (29) disposée sur la surface externe du composant (3, 4, 7-9, 13, 27), dans lequel la prise (29) est configurée pour :
recevoir une fiche (28) et/ou une connexion de pont (35) de telle sorte que l'au moins une interface électrique tangible (30, 32, 35, 36) soit dans la condition de court-circuit.

13. Le composant (3, 4, 7-9, 13, 27) selon l'une des revendications 1 à 12, dans lequel le composant (3, 4, 7-9, 13, 27) comprend au moins l'un parmi :
- un ventilateur (3),
- un actionneur du ventilateur (3),
- un amortisseur (4),
- un actionneur de l'amortisseur (4),
- une vanne (7-9),
- un actionneur de l'une des vannes (7-9),
- un capteur de débit (13),
- un capteur de débit massique (13),
- un capteur de gaz de combustion (27).

14. Le composant (3, 4, 7-9, 13, 27) selon l'une des revendications 3 à 9, dans lequel le composant (3, 4, 7-9, 13, 27) comprend au moins un contrôleur, dans lequel l'au moins un contrôleur du composant (3, 4, 7-9, 13, 27) se connecte en communication avec l'au moins un circuit de commutation (29, 37-61), dans lequel l'au moins un contrôleur du composant (3, 4, 7-9, 13, 27) est configuré pour :
lire le signal de sortie logique à partir de l'au moins une porte NOT ;
traiter le signal de sortie logique ; et
si le signal de sortie logique lu à partir de l'au moins une porte NOT indique un signal logique LOW :
attribuer une première adresse de bus au composant (3, 4, 7-9, 13, 27) ;
si le signal de sortie logique lu à partir de l'au moins une porte NOT indique un signal logique HIGH :
attribuer une deuxième adresse de bus au composant (3, 4, 7-9, 13, 27), dans lequel la deuxième adresse de bus diffère de la première adresse de bus.

15. Appareil de combustion comprenant un contrôleur (16), un brûleur (1) et un consommateur de chaleur (2) en communication fonctionnelle avec le brûleur (1), l'appareil de combustion comprenant également au moins un composant (3, 4, 7-9, 13, 27) selon la revendication 14 ;
dans lequel l'au moins un composant (3, 4, 7-9, 13, 27) se connecte en communication avec le contrôleur (16) de l'appareil de combustion via l'au moins un premier fil de bus et via l'au moins un deuxième fil de bus.
